(19) Europäisches Patentamt
European Patent Office
Office européen des brevets

(11) **EP 4 714 349 A1**

(12) **EUROPEAN PATENT APPLICATION**

(43) Date of publication:
**25.03.2026 Bulletin 2026/13**

(21) Application number: **25197220.4**

(22) Date of filing: **21.08.2025**

(51) International Patent Classification (IPC):
**A61B 5/16** (2006.01)   **A61B 5/374** (2021.01)
**A61B 5/00** (2006.01)

(52) Cooperative Patent Classification (CPC):
**A61B 5/374; A61B 5/165; A61B 5/7257;** A61B 5/18

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **20.09.2024 CN 202411319967**

(71) Applicants:
• **Kingfar International Inc.**
  **Beijing 100085 (CN)**

• **Nanjing Kingfar Health Technology Inc.**
  **Nanjing, Jiangsu 211112 (CN)**

(72) Inventors:
• **ZHAO, Qichao**
  **Beijing, 100085 (CN)**
• **WANG, Qingju**
  **Beijing, 100085 (CN)**

(74) Representative: **Meyer-Dulheuer MD Legal Patentanwälte PartG mbB**
**Hanauer Landstr. 287-289**
**60314 Frankfurt am Main (DE)**

(54) **METHOD AND APPARATUS FOR REMOVING INDIVIDUAL DIFFERENCES IN PHYSIOLOGICAL SIGNALS, EDGE COMPUTING DEVICE AND MEDIUM**

(57) Provided are a method and an apparatus for removing individual differences in physiological signals, an edge computing device, and a storage medium. The method includes: acquiring physiological signals of a plurality of subjects, where the physiological signals are acquired by a physiological signal acquisition device when the plurality of subjects perform a same task; extracting frequency domain features based on the physiological signals, where the frequency domain features are configured to characterize frequency characteristics of the physiological signals; extracting cepstral features based on the frequency domain features; and normalizing the cepstral features to obtain cepstral features of the physiological signals with at least part of individual differences among the plurality of subjects removed. The method according to the present disclosure is beneficial to normalizing physiological signals, and removing individual differences in the physiological signals without restricting the data distribution of physiological signals, thereby having a wider range of applications.

```
┌─────────────────────────────────┐
│ Acquiring physiological signals │  ⟋ S11
│   of a plurality of subjects    │
└─────────────────────────────────┘
                │
                ▼
┌─────────────────────────────────┐
│ Extracting frequency domain     │  ⟋ S12
│ features based on the           │
│ physiological signals           │
└─────────────────────────────────┘
                │
                ▼
┌─────────────────────────────────┐
│ Extracting cepstral features    │  ⟋ S13
│ based on the frequency domain   │
│ features                        │
└─────────────────────────────────┘
                │
                ▼
┌─────────────────────────────────┐
│ Performing normalization        │  ⟋ S14
│ processing on the cepstral      │
│ features to obtain cepstral     │
│ features of the physiological   │
│ signals with at least part      │
│ of individual differences among │
│ the plurality of subjects       │
│ removed                         │
└─────────────────────────────────┘
```

FIG. 1

EP 4 714 349 A1

## Description

## TECHNICAL FIELD

The present disclosure relates to the field of physiological signal processing technologies, and in particular, to a method and an apparatus for removing individual differences in physiological signals, an edge computing device and a storage medium.

## BACKGROUND

Physiological signals are naturally generated by a human body, with significant individual difference in both value ranges and baselines across subjects. To extract common characteristics from multiple individuals, individual differences need to be removed. One way to remove individual differences is to normalize data, that is, unify signals of different individuals into a same measurement unit prior to subsequent analysis.

In the related art, a method for normalizing the physiological signals includes min-max normalization and Z-Score normalization, however, the min-max normalization method is more suitable for a case where the data distribution is relatively concentrated. When new data is added, the max value and the min value may need to be redefined. The Z-Score normalization method needs to assume that the data distribution obeys a normal distribution.

## SUMMARY

The present disclosure provides a method and an apparatus for removing individual differences in physiological signals, an edge computing device and a storage medium, which are beneficial to normalizing physiological signals, and removing individual differences of the physiological signals without restricting the data distribution of physiological signals, thereby having a wider range of applications.

In a first aspect, the present disclosure provides a method for removing individual differences in physiological signals, including: acquiring physiological signals of a plurality of subjects, where the physiological signals are acquired by a physiological signal acquisition device when the plurality of subjects perform a same task; extracting frequency domain features based on the physiological signals, where the frequency domain features are configured to characterize frequency characteristics of the physiological signals; extracting cepstral features based on the frequency domain features; and performing normalization processing on the cepstral features to obtain cepstral features of the physiological signals with at least part of individual differences among the plurality of subjects removed.

In a possible implementation, the frequency domain features include a power spectral density of the physiological signals, and said extracting frequency domain features based on the physiological signals includes: dividing the physiological signals into signals of a plurality of windows in a sliding window manner; extracting an autocorrelation function for signals of each window; and performing a fast Fourier transform on the autocorrelation function to obtain the power spectral density of the physiological signals.

In a possible implementation, said extracting cepstral features based on the frequency domain features includes: taking a logarithm of the power spectral density of the physiological signals; and performing an inverse Fourier transform to obtain the cepstral features.

In a possible implementation, said performing normalization processing on the cepstral features includes: performing normalization processing on the cepstral features using a cepstral mean normalization model. A training method of the cepstral mean normalization model includes: acquiring training data, where the training data include physiological data of multiple subjects, the physiological data are obtained by performing cepstral feature extraction on physiological signals acquired by the physiological signal acquisition device, and the physiological data of each subject includes signals of a plurality of segments; calculating a sum of means and a sum of variances of signals of all segments of each subject; calculating a first average mean and a first average standard deviation of each subject based on the sum of means and the sum of variances, where the first average mean is an average mean among different segments of each subject, and the first average standard deviation is an average standard deviation among different segments of each subject; and calculating a second average mean and a second average standard deviation based on the first average mean and the first average standard deviation, where the second average mean is an average mean of signals among all subjects, and the second average standard deviation is an average standard deviation of signals among all subjects.

In a possible implementation, said performing normalization processing on the cepstral features using a cepstral mean normalization model includes: normalizing the cepstral features based on the second average mean and the second average standard deviation that are obtained by training the cepstral mean normalization model, to obtain cepstral features of the physiological signals with at least part of individual differences among the plurality of subjects removed.

In a possible implementation, before said extracting frequency domain features based on the physiological signals, the method further includes: performing filtering processing and/or independent component analysis processing on the physiological signals, where the filtering processing includes at least one of high-pass filtering, low-pass filtering, and notch filtering. Additionally/alternatively, the physiological signals include at least one of electroencephalographic signals, electrocardiographic signals, electromyographic signals, and electrodermal activity signals. Additionally/alternatively, an acquisition process of the physiological signals includes: wearing or attaching, by the plurality of subjects, the physiological signal acquisition device, performing, by the plurality of subjects, the same task in different fatigue states, where the different fatigue states include at least two of mild fatigue, moderate fatigue, and severe fatigue; and acquiring, by the physiological signal acquisition device, the physiological signals.

In a possible implementation, the method further includes: determining a fatigue state of each of the plurality of subjects using a subjective scale and/or an objective physiological signal analysis, where the fatigue state includes the mild fatigue, the moderate fatigue, or the severe fatigue; and establishing a correspondence between the fatigue state of each of the plurality of subjects and a corresponding physiological signal in the acquired physiological signals.

In a second aspect, the present disclosure provides an apparatus for removing individual differences in physiological signals, including: an acquisition module configured to acquire physiological signals of a plurality of subjects, where the physiological signals are acquired by a physiological signal acquisition device when the plurality of subjects perform a same task; a first extraction module configured to extract frequency domain features based on the physiological signals, where the frequency domain features are configured to characterize frequency characteristics of the physiological signals; a second extraction module configured to extract cepstral features based on the frequency domain features; and a normalization module configured to perform normalization processing on the cepstral features to obtain cepstral features of the physiological signals with at least part of individual differences among the plurality of subjects removed.

In a third aspect, the present disclosure provides an edge computing device, including: a processor and a memory configured to store a computer program, where the processor, when running the computer program, is configured to implement the method for removing individual differences in physiological signals according to the first aspect.

In a fourth aspect, the present disclosure provides a computer-readable storage medium on which a computer program is stored. The computer program, when running on a computer, is configured to implement the method for removing individual differences in physiological signals according to the first aspect.

Compared with the related art, the present disclosure has at least the following technical effects: according to a method for removing individual differences in physiological signals provided by the present disclosure, the frequency domain features are extracted from the acquired physiological signals of the plurality of subjects, the cepstral features are extracted based on the frequency domain features, and the cepstral features are input into the cepstral mean normalization model for normalization. That is, the cepstral features input into the model is normalized based on the mean and standard deviation parameters obtained from the training of the cepstral mean normalization model by adopting a cepstral mean normalization technology, which helps to achieve the normalization of physiological signals and remove the individual differences in physiological signals. There is no need to restrict the data distribution of physiological signals, so that the application range is wider and the model has stronger generalization ability.

## BRIEF DESCRIPTION OF DRAWINGS

FIG. 1 is a schematic flowchart of a method for removing individual differences in physiological signals according to an embodiment of the present disclosure;
FIG. 2 is a schematic flowchart of a method for extracting cepstral features according to an embodiment of the present disclosure;
FIG. 3 is a schematic flowchart of a method for training a cepstral mean normalization model according to an embodiment of the present disclosure;
FIG. 4 is a schematic structural diagram of an apparatus for removing individual differences in physiological signals according to an embodiment of the present disclosure; and
FIG. 5 is a schematic structural diagram of an edge computing device according to an embodiment of the present disclosure.

## DESCRIPTION OF EMBODIMENTS

In embodiments of the present disclosure, unless otherwise specified, the character "/" herein generally indicates an "or" relationship between the associated objects. For example, A/B may represent A or B. It should be understood that the term "and/or" describes an associated relationship of associated objects, indicating that there may be three relationships, for example, A and/or B can indicate: only A, both A and B, and only B.

It should be noted that terms such as "first" and "second" in the embodiments of the present disclosure are merely used for distinguishing description, and cannot be understood as indicating or implying relative importance or implicitly indicating the number of indicated technical features, or cannot be understood as indicating or implying an order.

In the embodiment of the present disclosure, "at least one" means one or more, and "a plurality of" means two or more. Further, "at least one of the following items" or a similar expression thereof refers to any combination of these items, including any combination of a single item or plural items. For example, at least one of A, B, or C may represent: A, B, C, a combination of A and B, a combination of A and C, a combination of B and C, or a combination of A, B and C. Each of A, B, and C may be an element, or may be a set including one or more elements.

In the embodiments of the present disclosure, expressions such as "exemplary", "in some embodiments", "in another embodiment" are used to indicate an example, an illustration, or a description. Any embodiment or design described herein as an "exemplary" should not be construed as preferred or advantageous over other embodiments or designs. To be precise, the use of the term "exemplary" is intended to present concepts in a concrete manner.

In the embodiments of the present disclosure, "of", "relevant", and "corresponding" may sometimes be used interchangeably. It should be noted that when the distinction is not emphasized, the meanings to be expressed are consistent. In the embodiments of the present disclosure, communication and transmission may sometimes be used interchangeably. It should be noted that when the distinction between them is not emphasized, the meanings to be expressed are consistent. For example, the transmission may include sending and/or receiving, and may be a noun or a verb.

An individual's emotional state, cognitive load level when completing a task, physical and mental stress and fatigue may seriously affect their work attitude and work efficiency. With the development of cognitive neuroscience, psychophysiology and computer science, the use of physiological signals to monitor a state of the individual in real time has gradually come into the sight of researchers, which has had a huge impact on fields such as traffic human factors, aerospace, and industrial construction.

In the field of traffic human factors, it can not only be used in a driver assistance system to identify different braking intentions of a driver and perform braking operations based on a classification result to reduce braking distance, but also can be used in an intelligent cockpit of the driver to warn about the fatigue state and dangerous driving behaviors of the driver, thereby reducing the accident occurrence rate. In the field of aerospace, it can monitor the cognitive load of pilots during different phases such as takeoff, ascent, cruise, descent and landing of airplanes, to design a more reasonable operation console, and improve the driving safety of various pilots. In the field of industrial construction, it can be used to identify the stress of construction workers, which helps improve the safety, health, happiness and productivity of the workers.

When a person receives external stimulation or generates a certain emotional state, the excitation of a sympathetic nervous system can cause dilation and contraction of blood vessels in the skin, sweat gland secretion, and changes in the heart's pumping rhythm, thereby leading to changes in both electrodermal activity (EDA) and heart rate variability (HRV). The physiological signals such as electroencephalographic signals, electromyographic signals, electrodermal signals, electrocardiographic signals, respiratory rate, and blood pressure can reflect the physiological fatigue state of individuals.

The following is an explanation of the electroencephalographic signals and HRV signals.

Electroencephalographic signals: the Electroencephalographic signals originates from the synchronous discharge of a large number of neurons' postsynaptic membranes in the cerebral cortex, and can be divided into evoked electroencephalogram and spontaneous electroencephalogram according to whether receiving external stimuli. The former is evoked by external stimuli and has obvious time-locking and phase-locking properties. Event-related potentials (ERP) can be obtained through superposition to reflect the cognitive processing of the brain. The spontaneous electroencephalogram has a specific physiological rhythm and can be divided into five frequency bands: $\delta$, $\theta$, $\alpha$, $\beta$ and $\gamma$. Each frequency band has specific scalp distribution and physiological significance, and can be modulated by stimuli to produce increases or decreases in the energy of specific frequency bands, which can reflect the mental and physical state of individuals.

HRV signals: heart rate variability is used to assess the autonomic nervous regulation function of the heart by quantifying the variability of sinus rhythm. The sinus rhythm time series is derived from a QRS complex (ventricular depolarization complex) to QRS interval (also called RR interval, referring to a time interval between two consecutive R waves) sequence in an electrocardiogram (ECG), by extracting a heartbeat interval (also called NN interval, referring to a time interval between two consecutive normal sinus rhythms) from a normal sinus to a normal sinus. Relatively high-frequency changes in sinus rhythm reflect parasympathetic (vagus nerve) regulation, while slower changes reflect parasympathetic and sympathetic regulation as well as non-autonomic factors.

A method for normalizing the physiological signals in the related art includes min-max normalization and Z-Score normalization.

(1) min-max normalization, also referred to as range normalization, is a linear transformation of original data, mapping a result value to [0, 1], and the conversion function is as follows:

$$x_{new} = \frac{x - x_{min}}{x_{max} - x_{min}},$$

where $x_{max}$ is a maximum value of the sample data, and $x_{min}$ is a minimum value of the sample data.
Such normalization method is more applicable in situations where the values are relatively concentrated. However, if $x_{max}$ and $x_{min}$ are unstable, which easily causes the normalization results to be unstable, thereby making the subsequent use effect unstable. In actual use, empirical constant values can be used to replace $x_{max}$ and $x_{min}$, and when new data is added, changes in $x_{max}$ and $x_{min}$ may occur, requiring redefinition.

(2) Z-Score normalization is a data normalization of the mean and standard deviation of the original data. The processed data conform to a standard normal distribution, that is, the mean is 0, and the standard deviation is 1. The transformation function is:

$$x_{new} = \frac{x-\mu}{\sigma}.$$

The advantage of this normalization method is that the normalized data retains useful information in outliers, making the algorithm less sensitive to outliers. The disadvantage of this normalization method is that it assumes the data distribution obeys the normal distribution.

Based on the problems, an embodiment of the present disclosure provides a method for removing individual differences in physiological signals, which is beneficial to normalizing physiological signals, and removing individual differences of the physiological signals without restricting the data distribution of physiological signals, thereby having a wider range of applications.

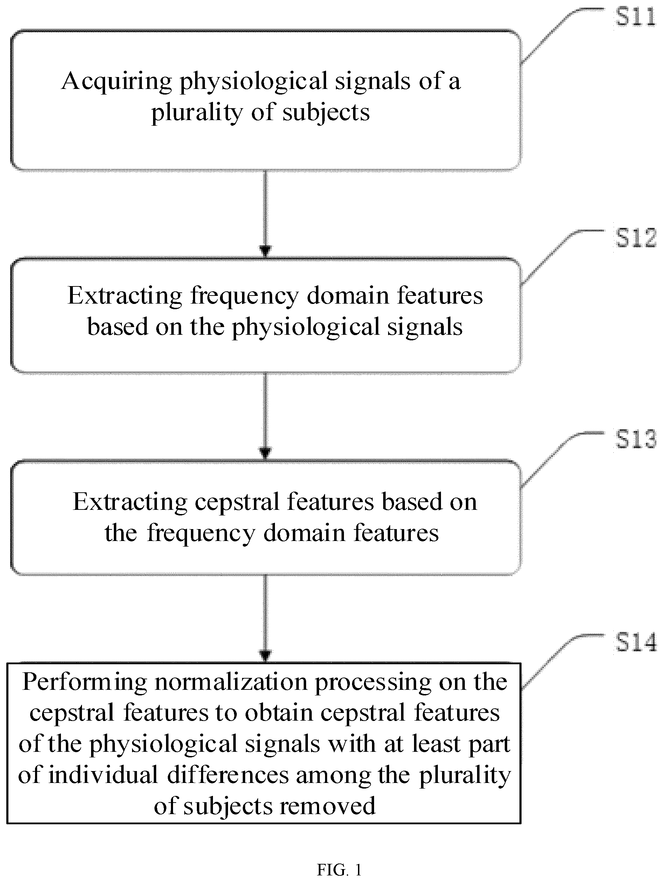

A method for removing individual differences in physiological signals according to an embodiment of the present disclosure will now be described with reference to FIG. 1 to FIG. 3.

FIG. 1 is a schematic flowchart of a method for removing individual differences in physiological signals according to an embodiment of the present disclosure, and the method includes step S11; step S12; step S13; and step S14.

Step S11, acquiring physiological signals of a plurality of subjects.

In this step, the plurality of subjects are designed to perform the same repetitive task under different fatigue states according to the research purpose, and the physiological signals are acquired by the physiological signal acquisition device worn or attached by the plurality of subjects. For example, in order to study the fatigue state of the driver, the plurality of subjects can be designed to perform a task of simulating the driving behavior under at least two of the states of mild fatigue, moderate fatigue and severe fatigue. A portable electroencephalograph with dry electrodes (or a wet electrode or a water electrode) can be used to collect electroencephalogram (EEG) signals of a plurality of drivers in different fatigue states, and the sampling rate can be 256Hz. The physiological signals are transmitted to an edge computing device for analysis and research. The edge computing device may be a desktop computer, a tablet computer, a notebook computer, a palmtop computer, an ultra-mobile personal computer (UMPC), a netbook, or the like, and the type of the edge computing device is not limited in the embodiments of the present disclosure.

In an example, the method according to the embodiments of the present disclosure can further include: determining a fatigue state of the subject using a subjective scale and/or an objective physiological signal analysis, where the fatigue state includes the mild fatigue, the moderate fatigue, or the severe fatigue; and establishing a correspondence between the fatigue state of the subject and the corresponding physiological signal in the acquired physiological signals.

After each task is performed, the subject fills in the subjective scale for subjective evaluation. That is, after completing a task, a self-assessment questionnaire can be presented to the subject to collect their subjective feelings during the task. For example, in a task of simulating driving behavior, the subject's self-evaluation of mental state is collected. The mental state may be alert, somewhat alert, neither alert nor drowsy, somewhat drowsy, drowsy, struggling to stay awake, or very drowsy. The subject fills in the questionnaire by themselves, the current fatigue state of the subject is assessed based on the questionnaire. For example, the fatigue state can be classified according to the mental state, where the mental states of alert, somewhat alert, and neither alert nor drowsy are classified as a non-fatigue state, the mental state of somewhat drowsy is classified as mild fatigue, the mental states of drowsy and struggling to stay awake are classified as moderate fatigue, and the mental state of very drowsy is classified as severe fatigue.

In an example, objective physiological signal analysis can also be adopted, that is, the current fatigue state of the subject is determined by collecting and analyzing the subject's physiological signals. The subjective scale and the objective physiological signal analysis can be used separately or in combination. After the current fatigue state of the subject is determined, the correspondence between the fatigue state and the physiological signals can be marked in the collected physiological signals, so that a cepstral feature normalization model can be trained on datasets under different fatigue states, thereby improving the accuracy and generalization of cepstral feature normalization.

In other embodiments, performing the same task can also be at least one of a stress tolerance test task, a bimanual coordination task, and a motion tracking task. The stress tolerance test task is configured to test the reaction ability of the subject, for example, pressing a corresponding color key or stepping on a corresponding pedal according to a screen prompt. Operational tasks, such as the bimanual coordination task, and the motion tracking task, are configured to test the coordination ability, bilateral arm stability, and hand flexibility of the subject. For example, when performing the bimanual coordination task, the subject can manipulate a joystick to control a ball moving within a track. For another example, when performing the motion tracking task, the subject can manipulate a joystick to control a crosshair to aim at a target. The crosshair is a part of an aiming means, and the crosshair is usually located at an upper end of the aiming means. The crosshair can be in the shape of a cylinder, triangle, rectangle and the like, and constitutes an aiming baseline in combination with the sight scale and notch. Physiological signals are acquired by the physiological signal acquisition device during the process where a plurality of subjects perform the same task. The physiological signals can include: photoplethysmogram (PPG) signals, electrodermal activity (EDA) signals, respiratory (RESP) signals, skin temperature (SKT) signals, peripheral capillary oxygen saturation (SpO2) signals, electrocardiographic (electrocardiogram, hereinafter referred to as ECG) signals, electromyogram (EMG, i.e., electromyographic signals), and human nine-axis posture data.

Step S12, extracting frequency domain features based on the physiological signals.

In an example, the frequency domain features are features extracted from the physiological signals in a frequency domain perspective, which can include a power spectral density, a spectral distribution, and the like, and are used to characterize frequency characteristics of the physiological signals. In other embodiments, extracting frequency domain features from the physiological signals may further include extracting features of multiple frequency bands. For example, for EEG signals, features within specific frequency bands may be extracted from the EEG signals, such as extracting features of five frequency bands: $\delta$(1-4Hz), $\theta$(4-8Hz), $\alpha$(8-12Hz), $\beta$ (12-31Hz) and $\gamma$(31-75Hz). The extraction of features of the frequency bands can help identify specific patterns or behaviors in the signals. For example, in EEG analysis, features of different frequency bands reflect electrical activity patterns of the brain in different states. For example, $\alpha$ waves are usually associated with relaxation and eyes-closed states, while $\beta$ waves are associated with alertness and attention. It should be noted that the division of frequency bands herein is not unique, and the number and types of extracted features of the frequency bands can be selected according to research purposes, which is not limited in the present disclosure. By extracting features of multiple frequency bands from the physiological signals, the physiological signals to be normalized are more targeted and include more useful information for subsequent research.

In one possible embodiment, before the step S12, the method according to the present disclosure further includes: performing filtering processing and/or independent component analysis processing on the physiological signals. In an example, the filtering processing includes at least one of high-pass filtering, low-pass filtering, and notch filtering. The high-pass filtering can be set to 0.5Hz, the low-pass filtering can be set to 70Hz, and the notch filtering can be set to 50Hz.

Filtering is mainly used to remove unwanted frequency components in the physiological signals to extract useful signal features or reduce the impact of noise. The high-pass filtering is configured to remove low-frequency noise, such as drift and baseline fluctuation. For example, in electroencephalogram (EEG) signal processing, the high-pass filtering can remove low-frequency components generated by eye tracking or ECG interference. The low-pass filtering is configured to remove high-frequency noise, such as electromyographic interference or power line interference. In electrocardiogram (ECG) signal processing, the low-pass filtering can remove high-frequency noise and artifacts. The notch filtering is dedicated to removing interference at specific frequencies, such as 50Hz or 60Hz power line interference in EEG signals. The selection of specific filtering methods needs to consider the characteristics of the signal and the type of noise. For example, if the signal mainly includes a low-frequency component, a low-pass filter may be required. For periodic interference, notch filtering needs is required.

Independent component analysis is used to separate statistically independent source signals from multivariate signals. It is mainly used in physiological signal processing to separate different physiological sources in mixed signals, such as separating brain electrical activity and other bioelectrical interference in EEG signals. The independent component analysis is suitable for multi-channel data and can effectively separate independent sources from multi-dimensional signals.

When the frequency ranges of the noise of the physiological signals are known and these frequency ranges are obviously different from the useful frequency ranges of the signals, or when specific frequency interference, such as power line interference, needs to be removed, the filtering needs to be performed on the physiological signals. When multiple independent sources are mixed in the physiological signals, and the statistical characteristics of these sources are different, or when specific physiological or non-physiological components need to be separated from the multi-channel physiological signals, such as separating the electrooculogram and electrocardiogram components in the EEG signals, the independent component analysis needs to be performed on the physiological signals.

In an example, when the physiological signals are electroencephalographic signals, high-pass filtering, low-pass filtering and notch filtering need to be performed on the electroencephalographic signals, which can effectively eliminate interference such as mains electricity and motion artifacts. The independent component analysis is also required, and artifact components such as electrooculogram and electromyogram in the electroencephalographic signals can be removed through the independent component analysis. By performing filtering processing and/or independent component analysis processing on the physiological signals, interference signals can be removed, and the normalization effect of the physiological signals is improved.

Step S13, extracting cepstral features based on the frequency domain features.

In this step, the frequency domain features include a power spectral density (PSD, hereinafter referred to as power spectrum) of the physiological signals. The power spectrum can be used to describe the distribution of signal power at different frequencies. Therefore, in the present disclosure, the frequency domain features can be extracted by calculating the power spectrum. Wiener-Khinchin theorem proves that the power spectrum of a signal is equal to the Fourier transform of the autocorrelation function of the signal, so there are two methods to calculate the power spectrum: 1. (square of the Fourier transform)/(interval length); 2. Fourier transform of the autocorrelation function. These two methods are respectively called a direct method and a correlation function method. Since the correlation function method has a better noise suppression effect and a smoother graph, the autocorrelation function method is used to calculate the power spectrum in the present disclosure.

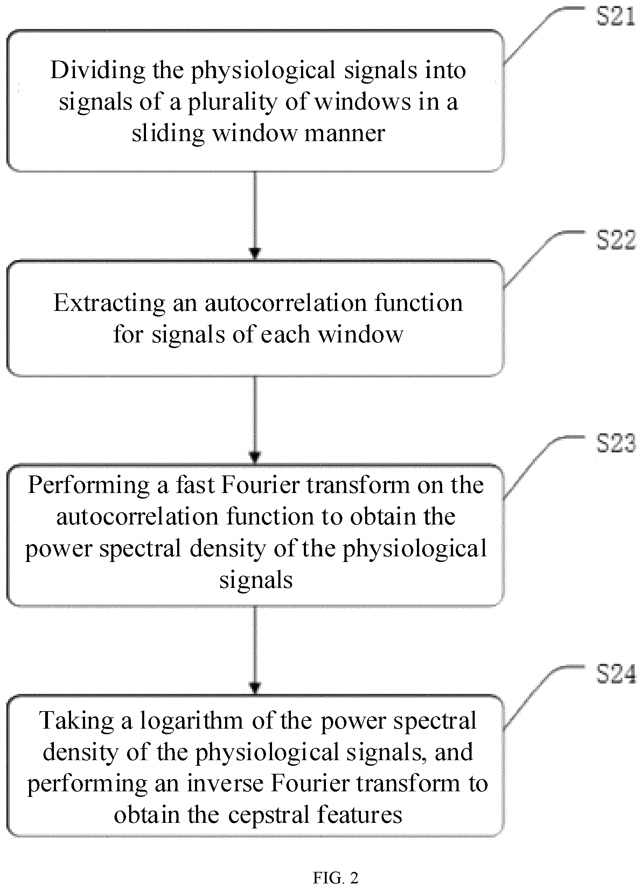

As shown in FIG. 2, which is a schematic flowchart of a method for extracting cepstral features according to an embodiment of the present disclosure, the extracting cepstral features includes step S21; step S22; step S23; and step S24.

Step S21, dividing the physiological signals into signals of a plurality of windows in a sliding window manner.

In an example, in the sliding window manner, the physiological signals are divided into a plurality of signals with 4 seconds as one window. It can be understood that the duration of the window can also be 2 seconds, 3 seconds, or the like, and the duration of the window is not limited in the present disclosure.

Step S22, extracting an autocorrelation function for signals of each window.

In an example, the autocorrelation function is extracted from the signals of each window. The autocorrelation function is usually defined as an expected value of the product of a signal and itself at different time delays. Taking the signal length of each window as 4 seconds and the original sampling rate as 256Hz as an example, the length of the extracted data is 256*4=1024 points, that is, each window includes 1024 sample points.

Step S23, performing a fast Fourier transform on the autocorrelation function to obtain the power spectral density of the physiological signals.

In an example, the Fast Fourier Transform (FFT) is performed on the autocorrelation function of each window to convert a time domain signal into a frequency domain signal, so as to obtain a frequency domain representation of the window.

It should be noted that the above steps S21-S23 are used to calculate the power spectrum using an autocorrelation function method.

Step S24, taking a logarithm of the power spectral density of the physiological signals, and performing an inverse Fourier transform to obtain the cepstral features.

In an example, taking a logarithm of the power spectral density of the physiological signals helps to enhance the frequency characteristics of the signals and can reduce the dynamic range difference between different frequency components. An inverse Fourier transform is performed on the logarithmic frequency domain data to obtain cepstral features. The cepstrum is a result of nonlinear transformation of a frequency domain of a signal, and is usually used in certain specific applications in signal processing, such as feature extraction. After the above steps S21-S24, each window can obtain a 1024-dimensional cepstral feature vector.

Step S14, performing normalization processing on the cepstral features to obtain cepstral features of the physiological signals with at least part of individual differences among the plurality of subjects removed.

The cepstral features refer to signal features obtained by performing logarithmic transformation and inverse Fourier transform on a power spectrum of a signal. These features can capture periodicity or frequency distribution characteristics of the signal, reveal periodic components and linear prediction characteristics of the signal, reduce redundant information in the spectrum, and retain important features of the signal, which helps to extract useful information from complex signals. The cepstral features are used for normalizing the physiological signals, thereby improving the efficiency and accuracy of normalization. The normalization is to scale these cepstral features to a uniform scale to remove individual differences among different subjects, that is, to remove individual differences in the physiological signals, so that during the subsequent comparison and analysis of the physiological signals, individual differences of the subjects can be excluded, and important features with relatively commonality among different subjects in the physiological signals can be focused on, such as periodic changes in frequency.

The normalized cepstral features can be applied to the field of multi-modal physiological signal processing. For example, the normalized cepstral features can be combined with visual information to improve the accuracy of emotion recognition through a multi-level fusion method. The normalized cepstral features can also be fused with features of other physiological signals at the feature level to improve the performance and robustness of the recognition system. In the classification stage, the normalized cepstral features can be fused with other modal information at the decision level to realize more accurate classification and recognition.

Therefore, the normalized cepstral features play multiple roles in the field of physiological signal processing, which not only helps to improve the accuracy of signal processing, but also enhance the robustness and anti-noise ability of the system. The performance of the physiological signal processing system can be further improved by fusing the normalized cepstral features with the multi-modal features.

According to the present disclosure, the cepstral features of the physiological signals are normalized to obtain normalized cepstral features, that is, to obtain the cepstral features of the physiological signals with at least part of individual differences among multiple subjects removed. These normalized cepstral features can be used for subsequent comparison and analysis of the physiological signals.

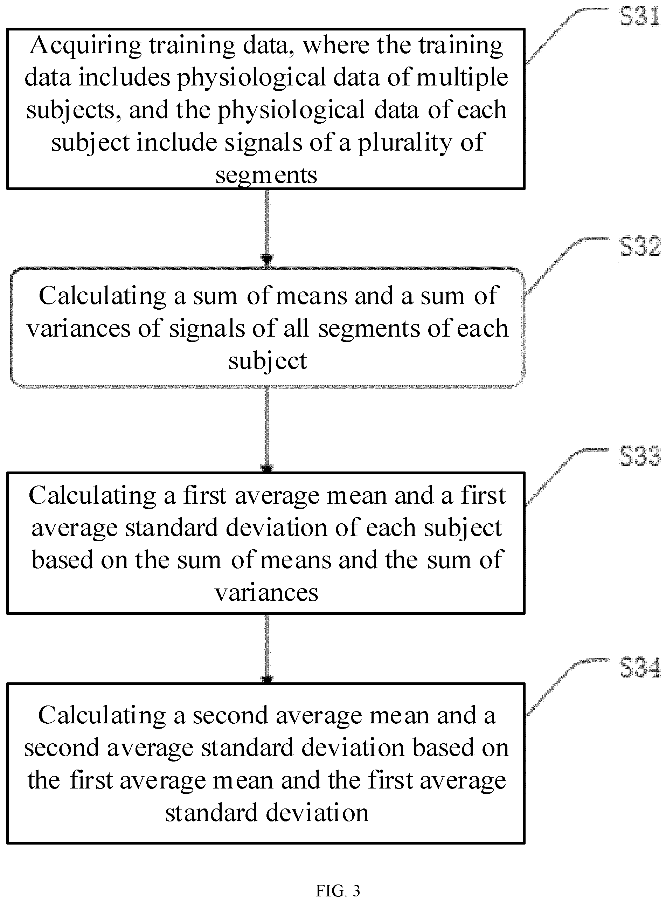

In this step, normalizing the performing normalization processing on the cepstral features includes: normalizing the cepstral features by a cepstral mean normalization model. A training method of the cepstral mean normalization model is shown in FIG. 3, which specifically includes step S31; step S32; step S33; and Step S34.

Step S31, acquiring training data. The training data include physiological data of multiple subjects, and the physiological data of each subject include signals of a plurality of segments.

In an example, the physiological signal acquisition device is configured to acquire physiological signals of multiple subjects, and extract the cepstral features of the physiological signals to obtain physiological data as training data for the model. The training data includes the physiological data of multiple subjects, and the physiological data of each subject includes signals of the plurality of segments. In an example, a 3-second window and a 1-second window step can be used, and finally a 3-second signal segment is generated every 1 second.

The following describes model training using an EEG signal as an example. It can be understood that different types of physiological signals and different data have different feature dimensions, and the model supports the input of physiological signals with multiple feature dimensions.

For example, the training data include EEG signals of multiple subjects (physiological data obtained after cepstral feature extraction), and the EEG signals of each subject include signals of different segments. The file format of the data can be: sub1: eeg1.csv, eeg2.csv, eeg3.csv; sub2: eeg4.csv, eeg5.csv; sub3: eeg6.csv, eeg7.csv; ......, where sub1 represents a subject 1, sub2 represents a subject 2, sub3 represents a subject 3, eeg1.csv, eeg2.csv and eeg3.csv represent EEG signals of three segments of the subject 1, eeg4.csv and eeg5.csv represent EEG signals of two segments of the subject 2, and eeg6.csv and eeg7.csv represent EEG signals of two segments of the subject 3.

Generally, during the data acquisition stage, the training data can be grouped according to different subjects. If grouping information is not provided, the training data can be grouped using a clustering algorithm, or EEG signals of each segment can be used as the physiological signals of one subject.

Step S32, calculating a sum of means and a sum of variances of signals of all segments of each subject.

At the beginning of model training, the number of segments, the sum of means, and the sum of variances for each subject are initialized, and the number of segments is the number of segments of the EEG signals. All training data are traversed, and for each subject, the sum of means and the sum of variances of signals of all segments for the subject are respectively calculated.

Step S33, calculating a first average mean and a first average standard deviation of each subject based on the sum of means and the sum of variances.

In an example, the first average mean is an average mean among different segments of each subject, and the first average standard deviation is an average standard deviation among different segments of each subject. The first average mean of the subject = the sum of means of the subject/ the number of segments of the subject, and the first average standard deviation of the subject = the square root of (the sum of variances of the subject/ the number of segments of the subject - the first average mean of the subject * the first average mean of the subject). The subject here refers to the same subject.

For example, the sum of means of the subject 1 is A1, the sum of variances of the subject 1 is B1, the first average mean of the subject 1 is a1, the first mean standard deviation of the subject 1 is b1, and the number of segments of the subject 1 is 5, then:

$$ a1 = \frac{A1}{5} \text{ , and } b1 = \sqrt{\left(\frac{B1}{5} - a1 * a1\right)}. $$

Step S34, calculating a second average mean and a second average standard deviation based on the first average mean and the first average standard deviation.

In an example, the second average mean is an average mean of signals among all subjects, and the second average standard deviation is an average standard deviation of signals among all subjects. The second average mean = the sum of the first average means of all subjects/the number of subjects, and the second average standard deviation = the sum of the first average standard deviations of all subjects/the number of subjects.

For example, the training data include data of three subjects, a first average mean of each subject is respectively a11, a12, and a13, a first average standard deviation of each subject is respectively b11, b12, and b13, a second average mean is a2, and a second average standard deviation is b2, then:

$$ a2 = \frac{(a11+a12+a1\ )}{3}, \text{ and } b2 = \frac{(b11+b12+b1\ )}{3}. $$

In an example, the performing normalization processing on the cepstral features using a cepstral mean normalization model specifically includes: normalizing the cepstral features based on the second average mean and the second average standard deviation obtained by training the cepstral mean normalization model, to obtain normalized cepstral features, that is, to obtain cepstral features of the physiological signals with at least part of individual differences among the plurality of subjects removed.

In an embodiment, two feature vectors $\mu$ and $\lambda$ can be obtained through model training, where $\mu$ represents the second average mean and $\lambda$ represents the

square of the second standard deviation mean. The specific implementation method for normalizing the cepstral features using the cepstral mean normalization model can include:

Normalizing the cepstral features according to the formula $y = \frac{x-\mu}{\lambda}$ , where x represents cepstral features input to the cepstral mean normalization model, and y represents a normalization result output by the cepstral mean normalization model.

According to the present disclosure, the frequency domain features are extracted from the acquired physiological signals of multiple subjects, the cepstral features are extracted based on the frequency domain features, and the cepstral features are input into the cepstral mean normalization model for normalization. That is, the cepstral features input into the model is normalized based on the mean and standard deviation parameters obtained from the training of the cepstral mean normalization model by adopting the cepstral mean normalization technology, which helps to achieve the normalization of physiological signals and remove the individual differences in physiological signals. There is no need to restrict the data distribution of physiological signals, so that the application range is wider and the model has stronger generalization ability.

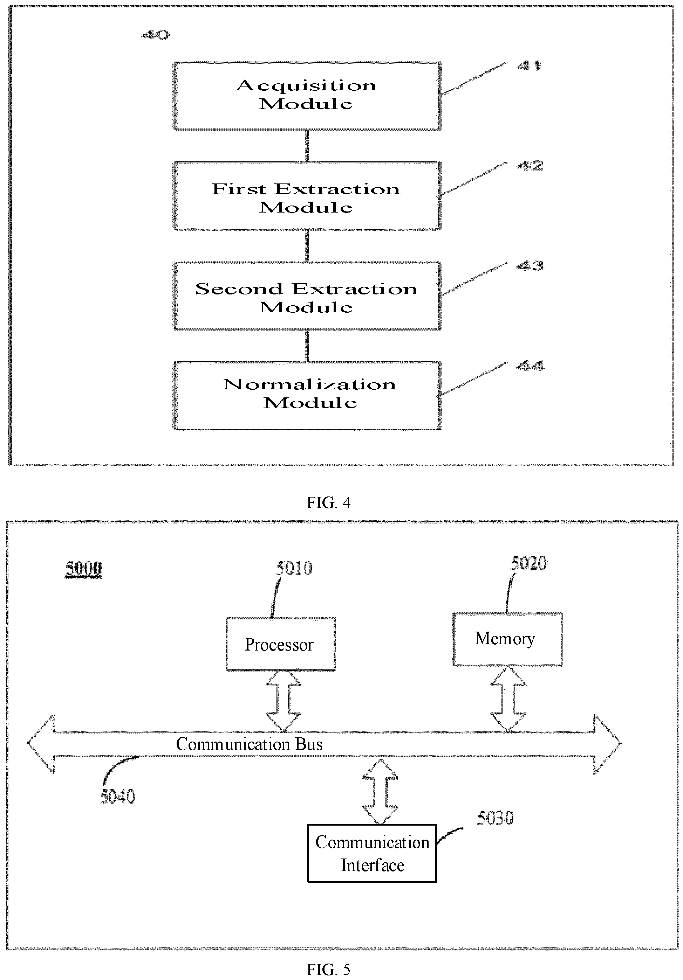

In other embodiments, the present disclosure further provides an apparatus for removing individual differences in physiological signals. As shown in FIG. 4, the apparatus 40 for removing individual differences in physiological signals may include an acquisition module 41; a first extraction module 42; a second extraction module 43; and a normalization module 44.

The acquisition module 41 is configured to acquire physiological signals of a plurality of subjects, where the physiological signals are acquired by a physiological signal acquisition device when the plurality of subjects perform a same task.

The first extraction module 42 is configured to extract frequency domain features based on the physiological signals, where the frequency domain features are used to characterize frequency characteristics of the physiological signals.

The second extraction module 43 is configured to extract cepstral features based on the frequency domain features.

The normalization module 44 is configured to perform normalization processing on the cepstral features to obtain cepstral features of the physiological signals with at least part of the individual differences among the plurality of subjects removed.

In a possible implementation, the frequency domain features include a power spectral density of the physiological signals, and the first extraction module 42 is further configured to: divide the physiological signals into signals of a plurality of windows in a sliding window manner; extract an autocorrelation function for signals of each window; and perform a fast Fourier transform on the autocorrelation function to obtain the power spectral density of the physiological signals.

In a possible implementation, the second extraction module 43 is further configured to take a logarithm of the power spectral density of the physiological signals, and perform an inverse Fourier transform to obtain the cepstral features.

In a possible implementation, the normalization module 44 is configured to perform normalization processing on the cepstral features, which includes: the normalization module 44 is configured to normalize the cepstral features using a cepstral mean normalization model, where a training method of the cepstral mean normalization model includes: acquiring training data, where the training data include physiological data of a plurality of subjects, the physiological data are obtained by performing cepstral feature extraction on the physiological signals acquired by the physiological signal acquisition device, and the physiological data of each subject include signals of a plurality of segments; calculating a sum of means and a sum of variances of signals of all segments of each subject; calculating a first average mean and a first average standard deviation of each subject based on the sum of means and the sum of variances, where the first average mean is an average mean among different segments of each subject, and the first average standard deviation is an average standard deviation among different segments of each subject; and calculating a second average mean and a second average standard deviation based on the first average mean and the first average standard deviation, where the second average mean is an average mean of signals among all subjects, and the second average standard deviation is an average standard deviation of signals among all subjects.

In a possible implementation, the normalization module 44 is configured to perform normalization processing on the cepstral features, which includes: the normalization module 44 is configured to: normalize the cepstral features according to the second average mean and the second average standard deviation that are obtained by training the cepstral mean normalization model, to obtain cepstral features of the physiological signals with at least part of individual differences among the plurality of subjects removed.

In a possible implementation, before the first extraction module 42 is configured to extract frequency domain features based on the physiological signals, the method further includes: performing filtering processing and/or independent component analysis processing on the physiological signals, where the filtering processing includes at least one of high-pass filtering, low-pass filtering, and notch filtering.

In a possible implementation, the physiological signals include at least one of electroencephalographic signals, electrocardiographic signals, electromyographic signals, and electrodermal activity signals.

In a possible implementation, an acquisition process of the physiological signals includes: wearing or attaching, by the plurality of subjects, the physiological signal acquisition device; performing, by the plurality of subjects, a same task in different fatigue states, where the different fatigue states include at least two of mild fatigue, moderate fatigue, and severe fatigue; and acquiring, by the physiological signal acquisition device, the physiological signals.

In a possible embodiment, the method further includes: determining a fatigue state of each of the plurality of subjects using a subjective scale and/or an objective physiological signal analysis, where the fatigue state includes the mild fatigue, the moderate fatigue, or the severe fatigue; and establishing a correspondence between the fatigue state of each of the plurality of subjects and the corresponding physiological signal in the acquired physiological signals.

The apparatus 40 for removing individual differences in physiological signals provided in the embodiments shown in FIG. 4 can be configured to implement the technical solutions of the method embodiments shown in the present disclosure, and for an implementation principle and a technical effect thereof, reference may be further made to related descriptions in the method embodiments.

It should be understood that the division of each module of the apparatus 40 for removing individual differences in physiological signals shown in FIG. 4 is merely a logical function division, and may be completely or partially integrated into one physical entity in actual implementation, or may be physically separated. Further, these modules may all be implemented in a form of software called by a processing element, or these modules may all be implemented in a form of hardware, or some modules may be implemented in a form of software called by a processing element, and some modules are implemented in a form of hardware. For example, the acquisition module can be a separate processing element, or can be integrated into a chip of the edge computing device for implementation. The other modules are implemented similarly. Further, all or part of these modules may be integrated together, or may be separately implemented. In an implementation process, each step of the above method or each module may be completed through an integrated logic circuit of hardware in a processor element or an instruction in a form of software.

For example, the above modules may be one or more integrated circuits configured to implement the above methods, such as: one or more application specific integrated circuits (hereinafter referred to as ASIC), or one or more digital signal processors (hereinafter referred to as DSP), or one or more field programmable gate arrays (hereinafter referred to as FPGA), or the like. For another example, these modules may be integrated together and implemented in a form of a system-on-a-chip (hereinafter referred to as SOC).

In the above embodiments, the involved processor may include, for example, a central processing unit (CPU), a microcontroller, or a digital signal processor (DSP), and may also include a graphics processing unit (GPU), an embedded neural-network process units (hereinafter referred to as NPU), and an image signal processing (hereinafter referred to as ISP). The processor may further include necessary hardware accelerators or logic processing hardware circuits, such as ASIC, or one or more integrated circuits for controlling the execution of the program of the technical solutions of the present disclosure. Further, the processor may have the function of operating one or more software programs that may be stored in the storage medium.

An embodiment of the present disclosure further provides a computer-readable storage medium on which a computer program is stored. The computer program, when running on a computer, is configured to implement the method provided in the embodiments of the present disclosure.

An embodiment of the present disclosure further provides a computer program product, which includes a computer program. The computer program, when running on a computer, is configured to implement the method according to the embodiments of the present disclosure.

An exemplary edge computing device provided in the embodiments of the present disclosure is further described with reference to FIG. 5, which is a schematic structural diagram of an edge computing device 5000.

The edge computing device 5000 may include: at least one processor; and at least one memory communicatively connected to the processor. The memory stores program instructions executed by the processor, and the processor can execute the method for removing individual differences in physiological signals provided by the embodiments of the present disclosure by calling the above program instructions.

FIG. 5 shows a block diagram of an exemplary edge computing device 5000 suitable for implementing embodiments of the present disclosure. The edge computing device 5000 shown in FIG. 5 is merely an example, and has no limitation to the function and scope of use of the embodiments of the present disclosure.

As shown in FIG. 5, the edge computing device 5000 is represented in a form of a general-purpose computing device. Components of the edge computing device 5000 may include, but are not limited to: one or more processors 5010, a memory 5020, a communication bus 5040 connecting different system components (including the memory 5020 and the processor 5010), and a communication interface 5030.

In an optional example, the edge computing device is deployed on an edge side, and is deployed in communication and connection with a terminal such as a signal acquisition device or is deployed integrally with the terminal. The edge computing device obtains the physiological signals collected by the signal acquisition device and performs individual difference elimination processing (that is, normalization processing), and performs subsequent processing such as state classification based on the processed signals, or the edge computing device may transmit the processed signals to a server or a cloud for subsequent processing such as state classification. According to the present disclosure, at least part of the computing tasks is migrated to an edge device, which can leverage the advantages of edge computing, improve data processing efficiency, reduce the latency and network resource occupation and reliance, and also facilitate data security and privacy protection.

The communication bus 5040 represents one or more of several types of bus structures, including a memory bus or a memory controller, a peripheral bus, a graphics acceleration port, a processor, or a local bus using any of a variety of bus structures. For example, these architectures include but are not limited to industry standard architecture (hereinafter referred to as ISA) bus, micro channel architecture (hereinafter referred to as MAC) bus, enhanced ISA bus, video electronics standards association (hereinafter referred to as VESA) local bus, and peripheral component interconnect (hereinafter referred to as PCI) Bus.

The edge computing device 5000 typically includes a variety of computer system readable media. These media may be any available media that can be accessed by the edge computing device, including volatile and non-volatile media, removable and non-removable media.

The memory 5020 may include a computer system readable medium in a form of a volatile memory, such as a random-access memory (hereinafter referred to as RAM) and/or a cache memory. The edge computing device may further include other removable/non-removable, and volatile/non-volatile computer system storage media. Although not shown in FIG. 5, a disk driver for reading and writing a removable non-volatile disk (such as a "floppy disk"), and an optical disk driver for reading and writing a removable non-volatile optical disk (such as compact disc read only memory (hereinafter referred to as CD-ROM), digital video disc read only memory (hereinafter referred to as DVD ROM), or other optical media) can be provided. In these cases, each driver may be connected to communication bus 5040 via one or more data media interfaces. The memory 5020 may include at least one program product having a set (e.g., at least one) of program modules configured to perform functions of the embodiments of the present disclosure.

A program/utility tool with a set (at least one) of program modules may be stored in the memory 5020, such program modules include, but not limited to, an operating system, one or more application programs, other program modules and program data, each or some combination of which may include an implementation of a network environment. The program modules generally perform functions and/or methods in the embodiments described in the present disclosure.

The edge computing device 5000 may also communicate with one or more external devices (such as a keyboard, a pointing device, a display, etc.), one or more devices that enable a user to interact with the edge computing device, and/or any device (such as a network card, a modem, etc.) that enables the edge computing device to communicate with one or more other computing devices. Such communication may be performed through the communication interface 5030. Further, the edge computing device 5000 may further communicate with one or more networks (such as a local area network (hereinafter referred to as LAN), a wide area network (hereinafter referred to as WAN) and/or a public network (such as the Internet)) through a network adapter (not shown in FIG. 5), which can communicate with other modules of the edge computing device through the communication bus 5040. It should be understood that although not shown in FIG. 5, other hardware and/or software modules can be used in conjunction with the edge computing device 5000, including but not limited to: a microcode, a device driver, a redundant processing unit, an external disk drive array, and a redundant array of independent drives (hereinafter referred to as RAID) system, a tape driver, and a data backup storage system.

By running a program stored in the memory 5020, the processor 5010 executes various functional applications and data processing, such as implement the method provided in the embodiments of the present disclosure.

Those skilled in the art may recognize that the units and algorithm steps described in the embodiments disclosed herein can be implemented using electronic hardware, and a combination of electronic hardware and computer software. Whether these functions are executed in hardware or software depends on the specific application and design constraints of the technical solutions. Those skilled in the art may use different methods to implement the described functions for each specific application, but such implementation should not be considered to be beyond a scope of the present disclosure.

Those skilled in the art should clearly understand that, for convenience and conciseness of description, the specific operating processes of the above-described system, apparatus and unit can refer to the corresponding process in the above method embodiments, which will not be repeated herein.

In some embodiments provided in the present disclosure, if any function is implemented in a form of a software function unit and is sold or used as an independent product, the function may be stored in a computer-readable storage medium. Based on such understanding, the technical solution of the present disclosure can essentially, or the part that contributes to the related art, or a part of the technical solution be embodied in the form of a software product. The computer software product is stored in the storage medium and includes a number of instructions for enabling a computer device, which may be a personal computer, a server, or a network device, etc., to execute all or some of the steps of the method described in each embodiment of the present disclosure.

The above storage medium includes: various media that may store program codes, such as universal serial bus (USB) flash drive, mobile hard disk, read-only memory (hereinafter referred to as ROM), random access memory (hereinafter referred to as RAM), magnetic disk, or optical disk.

The above descriptions are merely specific implementations of the present disclosure. Any person skilled in the art can easily think of changes or substitutions within the technical scope disclosed in the present disclosure, and they should be covered within the protection scope of the present disclosure. The protection scope of the present disclosure should be based on the protection scope of the claims.

**Claims**

1. A method for removing individual differences in physiological signals, comprising:

   acquiring physiological signals of a plurality of subjects, wherein the physiological signals are signals acquired by a physiological signal acquisition device when the plurality of subjects performs a same task;
   extracting frequency domain features based on the physiological signals, wherein the frequency domain features are configured to characterize frequency characteristics of the physiological signals;
   extracting cepstral features based on the frequency domain features; and
   performing normalization processing on the cepstral features to obtain cepstral features of the physiological signals with at least part of individual differences among the plurality of subjects removed.

2. The method according to claim 1, wherein the frequency domain features comprise a power spectral density of the physiological signals, and said extracting frequency domain features based on the physiological signals comprises:

   dividing the physiological signals into signals of a plurality of windows in a sliding window manner;
   extracting an autocorrelation function for signals of each window; and
   performing a fast Fourier transform on the autocorrelation function to obtain the power spectral density of the physiological signals.

3. The method according to claim 2, wherein said extracting cepstral features based on the frequency domain features comprises:
   taking a logarithm of the power spectral density of the physiological signals, and performing an inverse Fourier transform to obtain the cepstral features.

4. The method according to any one of claims 1 to 3, wherein said performing normalization processing on the cepstral features comprises:

   performing normalization processing on the cepstral features using a cepstral mean normalization model, and
   wherein a training method of the cepstral mean normalization model comprises:

   acquiring training data, wherein the training data comprise physiological data of multiple subjects, the physiological data are obtained by performing cepstral feature extraction on the physiological signals acquired by the physiological signal acquisition device, and the physiological data of each subject comprises signals of a plurality of segments;
   calculating a sum of means and a sum of variances of signals of all segments of each subject;
   calculating a first average mean and a first average standard deviation of each subject based on the sum of means and the sum of variances, wherein the first average mean is an average mean among different segments of each subject, and the first average standard deviation is an average standard deviation among different segments of each subject; and
   calculating a second average mean and a second average standard deviation based on the first average mean and the first average standard deviation, wherein the second average mean is an average mean of signals among all subjects, and the second average standard deviation is an average standard deviation of signals among all subjects.

5. The method according to claim 4, wherein said performing normalization processing on the cepstral features using a cepstral mean normalization model comprises:
   normalizing the cepstral features based on the second average mean and the second average standard deviation that are obtained by training the cepstral mean normalization model, to obtain cepstral features of the physiological signals with at least part of individual differences among the plurality of subjects removed.

6. The method according to claim 1, wherein before said extracting frequency domain features based on the physiological signals, the method further comprises:

performing filtering processing and/or independent component analysis processing on the physiological signals, wherein the filtering processing comprises at least one of high-pass filtering, low-pass filtering and notch filtering; and/or

wherein the physiological signals comprise at least one of electroencephalographic signals, electrocardiographic signals, electromyographic signals, and electrodermal activity signals; and/or

wherein an acquisition process of the physiological signals comprises:

wearing or attaching, by the plurality of subjects, the physiological signal acquisition device;

performing, by the plurality of subjects, the same task in different fatigue states, wherein the different fatigue states comprise at least two of mild fatigue, moderate fatigue, and severe fatigue; and

acquiring, by the physiological signal acquisition device, the physiological signals.

7. The method according to claim 6, further comprising:

determining a fatigue state of each of the plurality of subjects using a subjective scale and/or an objective physiological signal analysis, wherein the fatigue state comprises the mild fatigue, the moderate fatigue, or the severe fatigue; and

establishing a correspondence between the fatigue state of each of the plurality of subjects and a corresponding physiological signal in the acquired physiological signals.

8. An apparatus for removing individual differences in physiological signals, comprising:

an acquisition module configured to acquire physiological signals of a plurality of subjects, wherein the physiological signals are acquired by a physiological signal acquisition device when the plurality of subjects performs a same task;

a first extraction module configured to extract frequency domain features based on the physiological signals, wherein the frequency domain features are configured to characterize frequency characteristics of the physiological signals;

a second extraction module configured to extract cepstral features based on the frequency domain features; and

a normalization module configured to perform normalization processing on the cepstral features to obtain cepstral features of the physiological signals with at least part of the individual differences among the plurality of subjects removed.

9. The apparatus according to claim 8, wherein the frequency domain features comprise a power spectral density of the physiological signals, and the first extraction module is configured to extract frequency domain features based on the physiological signals, comprising:

dividing the physiological signals into signals of a plurality of windows in a sliding window manner;

extracting an autocorrelation function for signals of each window; and

performing a fast Fourier transform on the autocorrelation function to obtain the power spectral density of the physiological signals.

10. The apparatus according to claim 9, wherein the second extraction module is configured to extract cepstral features based on the frequency domain features, comprising:

taking a logarithm of the power spectral density of the physiological signals, and performing an inverse Fourier transform to obtain the cepstral features.

11. The apparatus according to any one of claims 8 to 10, wherein the normalization module is configured to perform normalization processing on the cepstral features, comprising:

performing normalization processing on the cepstral features using a cepstral mean normalization model, and

wherein the apparatus further comprises a training module, and the training module is configured to:

acquire training data, wherein the training data comprise physiological data of multiple subjects, the physiological data are obtained by performing cepstral feature extraction on the physiological signals acquired by the physiological signal acquisition device, and the physiological data of each subject comprises signals of a plurality of segments;

calculate a sum of means and a sum of variances of signals of all segments of each subject;

calculate a first average mean and a first average standard deviation of each subject based on the sum of means and the sum of variances, wherein the first average mean is an average mean among different segments of each subject, and the first average standard deviation is an average standard deviation among different segments of each subject; and

calculate a second average mean and a second average standard deviation based on the first average mean and the first average standard deviation, wherein the second average mean is an average mean of signals among all subjects, and the second average standard deviation is an average standard deviation of signals among all subjects.

12. The apparatus according to claim **11,** wherein the normalization module is configured to perform normalization processing on the cepstral features using a cepstral mean normalization model, comprising: normalizing the cepstral features based on the second average mean and the second average standard deviation that are obtained by training the cepstral mean normalization model, to obtain cepstral features of the physiological signals with at least part of individual differences among the plurality of subjects removed.

13. The apparatus according to claim 8, wherein the apparatus further comprises:

a preprocessing module configured to perform filtering processing and/or independent component analysis processing on the physiological signals before extracting frequency domain features based on the physiological signals, wherein the filtering processing comprises at least one of high-pass filtering, low-pass filtering and notch filtering, and/or

wherein the physiological signals comprise at least one of electroencephalographic signals, electrocardiographic signals, electromyographic signals, and electrodermal activity signals, and/or

wherein the apparatus further comprises:

an acquisition module, wherein the acquisition module is configured to wear or attach, by the plurality of subjects, the physiological signal acquisition device, perform, by the plurality of subjects, the same task in different fatigue states, and acquire, by the physiological signal acquisition device, the physiological signals, wherein the different fatigue states comprise at least two of mild fatigue, moderate fatigue, and severe fatigue; and

an establishment module, wherein the establishment module is configured to determine a fatigue state of each of the plurality of subjects using a subjective scale and/or an objective physiological signal analysis, wherein the fatigue state comprises the mild fatigue, the moderate fatigue, or the severe fatigue, and establish a correspondence between the fatigue state of each of the plurality of subjects and a corresponding physiological signal in the acquired physiological signals.

14. An edge computing device, comprising: a processor and a memory configured to store a computer program, wherein the processor, when running the computer program, is configured to implement the method for removing individual differences in physiological signals according to any one of claims 1-7.

15. A computer-readable storage medium on which a computer program is stored, wherein the computer program, when running on a computer, is configured to implement the method for removing individual differences in physiological signals according to any one of claims 1-7.

Acquiring physiological signals of a plurality of subjects — S11

Extracting frequency domain features based on the physiological signals — S12

Extracting cepstral features based on the frequency domain features — S13

Performing normalization processing on the cepstral features to obtain cepstral features of the physiological signals with at least part of individual differences among the plurality of subjects removed — S14

FIG. 1

Dividing the physiological signals into signals of a plurality of windows in a sliding window manner ⎯ S21

Extracting an autocorrelation function for signals of each window ⎯ S22

Performing a fast Fourier transform on the autocorrelation function to obtain the power spectral density of the physiological signals ⎯ S23

Taking a logarithm of the power spectral density of the physiological signals, and performing an inverse Fourier transform to obtain the cepstral features ⎯ S24

FIG. 2

Acquiring training data, where the training data includes physiological data of multiple subjects, and the physiological data of each subject include signals of a plurality of segments — S31

Calculating a sum of means and a sum of variances of signals of all segments of each subject — S32

Calculating a first average mean and a first average standard deviation of each subject based on the sum of means and the sum of variances — S33

Calculating a second average mean and a second average standard deviation based on the first average mean and the first average standard deviation — S34

FIG. 3

FIG. 4

FIG. 5

Europäisches
Patentamt

European
Patent Office

Office européen
des brevets

# EUROPEAN SEARCH REPORT

**Application Number**

EP 25 19 7220

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JUHA M KORTELAINEN ET AL: "Sleep Staging Based on Signals Acquired Through Bed Sensor", IEEE TRANSACTIONS ON INFORMATION TECHNOLOGY IN BIOMEDICINE, IEEE SERVICE CENTER, LOS ALAMITOS, CA, US, vol. 14, no. 3, 1 May 2010 (2010-05-01), pages 776-785, XP011345715, ISSN: 1089-7771, DOI: 10.1109/TITB.2010.2044797 * pages 776-779 * | 1-15 | INV. A61B5/16 A61B5/374 A61B5/00 |
| X | WO 2021/226778 A1 (UNIV ZHEJIANG [CN]) 18 November 2021 (2021-11-18) * pages 2, 8, 9 * | 1-15 | |
| X | CHEN KUN ET AL: "EEG-based mental fatigue detection using linear prediction cepstral coefficients and Riemann spatial covariance matrix", JOURNAL OF NEURAL ENGINEERING, IOP PUBLISHING, BRISTOL, GB, vol. 19, no. 6, 28 November 2022 (2022-11-28), XP020437138, ISSN: 1741-2560, DOI: 10.1088/1741-2552/ACA1E2 [retrieved on 2022-11-28] * pages 1, 6, 7 * | 1-15 | |
| X | CN 115 778 390 A (UNIV WUHAN TECH) 14 March 2023 (2023-03-14) * paragraphs [0005] - [0010], [0062] - [0067] * | 1-15 | |
| A | CN 113 679 396 A (BEIJING BRAIN UP TECH CO LTD) 23 November 2021 (2021-11-23) * the whole document * | 1-15 | |

**TECHNICAL FIELDS SEARCHED (IPC)**

A61B

The present search report has been drawn up for all claims

| Place of search | Date of completion of the search | Examiner |
|---|---|---|
| The Hague | 30 January 2026 | Vanderperren, Yves |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

......................................................................

& : member of the same patent family, corresponding
  document

EPO FORM 1503 03.82 (P4C01)

## ANNEX TO THE EUROPEAN SEARCH REPORT
## ON EUROPEAN PATENT APPLICATION NO.

EP 25 19 7220

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2026

| Patent document cited in search report | Publication date | Patent family member(s) | Publication date |
|---|---|---|---|
| WO 2021226778 A1 | 18-11-2021 | NONE | |
| CN 115778390 A | 14-03-2023 | NONE | |
| CN 113679396 A | 23-11-2021 | NONE | |

EPO FORM P0459

For more details about this annex : see Official Journal of the European Patent Office, No. 12/82